# EUROPEAN PATENT APPLICATION

(11) **EP 4 438 017 A1**
(43) Date of publication of application: **02.10.2024**
(21) Application number: 24166196.6
(22) Date of filing: 26.03.2024
(51) Int. Cl.: A61F 9/008

(54) **FLASH-BACK REDUCTION IN OPTHALMIC TREATMENT APPARATUS**

(30) Priority: 30.03.2023 US 202318193026
(71) Applicant: NORLASE ApS, 2750 Ballerup (DK)
(72) Inventor: CHRISTENSEN, Mathias, 2750 Ballerup (DK); SKOVGAARD, Peter, 3460 Birkerød (DK)
(74) Representative: Guardian IP Consulting I/S

(57) **Abstract**

A laser apparatus for providing a treatment laser beam for photothermal ophthalmic treatment comprising a treatment light source configured to generate a laser beam having a first target wavelength and having a first output power, an aiming light source configured to generate an aiming light beam having a second target wavelength and having a second output power lower than the first output power wherein the laser apparatus is configured to selectively suppress a set of wavelength components of the laser beam generated by the treatment light source to create a treatment laser beam, the set of wavelength components having wavelengths within a first and/or second suppressing wavelength range, the first and/or second suppressing wavelength ranges being located on respective sides of the first target wavelength, and wherein the laser apparatus is further configured to output the treatment laser beam.

## Description

### Technical Field

The present disclosure relates to a laser apparatus for ophthalmic treatment, in particular photothermal ophthalmic treatment. The present disclosure further relates to a laser apparatus, which minimizes or eliminates the amount of unwanted flash-back light emanating from a treatment laser reaching the treating health care provider.

### BACKGROUND OF THE INVENTION

Laser-based ophthalmic treatment, such as laser photocoagulation, is a well-established practice within ophthalmology for treating diseases such as glaucoma, diabetic retinopathy, wet age-related macular degeneration (AMD), and other retinal diseases.

Laser coagulation requires the exposure of the retina to visible laser light and relies on the selective absorption in melanin and hemoglobin, typically at a wavelength range of 514-580 nanometers (nm), ie. visible green light, to achieve blood vessel coagulation. Laser photocoagulators focus on this range of wavelengths and aim to provide small spot sizes, typically 50 to 500 µm, adjustable power, e.g. in the range 0-2.0 W, and long depth of focus to increase treatment precision and quality with less heat, to minimize patient discomfort and effects to surrounding tissue. In the course of treatments, physicians will regularly set and update parameters dictating the laser energy to be delivered. These parameters can include power, pulse duration, and laser spot size, among other examples.

Commonly, slit lamps are used for delivering the laser energy to the patient's eye. In these systems, the patients typically sit up in an examination chair, rest their chin on a chin rest, and place their forehead against a forehead band, both of which keep the patient's head in place during the procedure.

Another common device is a Laser Indirect Ophthalmoscope (LIO), which is a head mounted device, worn by the doctor to deliver laser energy into a patient's eye. During procedures using the LIO, the doctor moves around the patient to deliver the laser energy to the desired portions of the retina, where the patient is usually in a supine position.

Photothermal ophthalmic therapy typically involves directing a treatment beam, in particular a treatment laser beam, onto a location on a structure in the subject's eye, in particular the retina. The treatment beam must have a therapeutically effective power and wavelength, i.e. it must be configured for photocoagulation. Photothermal ophthalmic treatment is typically performed point-by-point (single spot delivery), where each individual dose of radiation is directed and delivered to a selected target position on the structure to be treated. The treatment beam may be individually directed to the respective selected treatment positions by a physician. To this end, an aiming laser beam at a visible wavelength different from the treatment wavelength, is typically used in conjunction with, in particular prior to, a treatment laser beam. The aiming laser beam, sometimes also referred to as a pilot beam, indicates (targets) the treatment location on the retina or other structure, which is subsequently to be treated by the treatment laser beam. Each dose at each treatment location is individually initiated by the physician, e.g. by activating a foot switch.

Point-by-point (single spot) treatment tends to be a lengthy procedure, in particular when a large number of treatment locations are needed. This increases treatment costs, may cause physician fatigue as well as discomfort for the patient being treated. Ultimately, this may cause a prematurely terminated treatment, in which case the patient is left under-treated or needs to return at a later date to finish treatment.

Several approaches have been suggested in an attempt to reduce treatment times. One such approach involves multi-spot treatment (pattern laser treatment) where the aiming light beam is controlled to indicate multiple treatment locations and where the physician can activate a sequence of doses, which are then automatically directed to multiple treatment locations.

To protect the doctor from the treatment laser beam inadvertently damaging the doctor's eyes, a laser safety filter is used. This safety filter is designed to block the coherent laser light of the treatment laser beam from reaching the doctor. The doctor views the eye of the patient through an ocular, and the safety filter is typically placed on the optical path between the patient's eye and the ocular. Since the doctor is viewing the retina through the safety filter, the filter should transmit as much light as possible to allow the doctor to have the clearest possible view of the retina and see small color variations on the retina. In technical terms, this means that the filter should have high color clarity or high color rendering index.

In addition, in order for the doctor to direct the treatment laser beam to the correct area of the retina, a usually red aiming laser beam is used, which must be clearly visible to the doctor. The power and wavelength of the aiming light beam is selected to not cause any damage to the doctor or patient.

Another problem encountered in ophthalmic treatment apparatuses making use of diode lasers to generate the treatment laser beam, is that many doctors are uncomfortable with any leaking of green light during the treatment. Such leaking green light is typically called flash-back. Such flash-back can, in principle, be dangerous if it is laser light at a sufficiently high power. Flash-back can be an indication that the safety filter is faulty or even missing, which is why doctors are often very observant about this. However, typically, the flash-back light is incoherent light at low power. For many diode lasers, a very small amount of spontaneously emitted light, adjacent in spectrum to the lasing wavelengths exists. The power level of this light is often as much 4-5 orders of magnitude below the laser power. Because of the incoherent nature of this light and the low power, such light is harmless to the human eye. However, even in case of such safe and harmless light, flash-back can be distracting and disturbing to some doctors.

### SUMMARY OF THE INVENTION

In view of the above, it remains desirable to provide a laser apparatus for ophthalmic treatment that mitigates one or more of the disadvantages of prior art systems or that at least can serve as an alternative.

According to a first aspect, disclosed herein is a laser apparatus for providing a treatment laser beam for photothermal ophthalmic treatment, the laser apparatus comprising:
- a treatment light source configured to generate a laser beam having a first target wavelength and having a first output power;
- an aiming light source configured to generate an aiming light beam having a second target wavelength and having a second output power lower than the first output power;
wherein the laser apparatus is configured to selectively suppress a set of wavelength components of the laser beam generated by the treatment light source to create a treatment laser beam, the set of wavelength components having wavelengths within a first and/or second suppressing wavelength range, the first and second suppressing wavelength ranges being located on respective sides of the first target wavelength, and wherein the laser apparatus is further configured to output the treatment laser beam.

By suppressing light within a wavelength range on either side of the target wavelength, unwanted light emanating from the treatment light source, will be prevented from reaching the doctor during treatment. Suppressing light in the context of this disclosure, should be understood to mean reducing the light by at least a factor of 2, but the light should preferably be suppressed by a factor of 5 or more. Such reduction will reduce the power to a level comparable with the white light used for illuminating the eye of the patient. At this low power level, the flash-back is no longer disturbing to the doctor.

In some embodiments, the treatment beam output power should be between 100 mW and 3 W. This output power range corresponds to the range in which the treatment laser beam will be therapeutically effective. The output power of the aiming light beam should be in the range 1 µW and 1 mW, where the aiming light beam will be visible to the doctor when looking at the patient's eye, but where the aiming light beam does not have enough output power to cause any damage or changes to the patient's eye or to the doctor.

In some embodiments, the treatment light source comprises one or more direct diode lasers for generating the laser beam provided/output by the treatment light source.

A high-power green treatment laser beam is emitted by the laser apparatus, and directed to the optical delivery system, which then directs the laser light through the pupil of the patient and focused on the retina. The laser power is sufficiently high to cause changes on the retina. During the treatment, the doctor is viewing the retina so that he can aim the laser beam to appropriate locations.

By making use of direct diode lasers instead of solid-state lasers, the laser apparatus will be cheaper to produce and can be made more compactly.

In some embodiments, the laser apparatus comprises an optical system configured for outputting the treatment laser beam for delivery towards the eye of a subject to be treated. In some embodiments, the optical system may be configured for manipulating and shaping the treatment beam and for delivering the treatment laser beam and the aiming light beam to an optical delivery fiber, which may then guide the light to an optical delivery system, which then directs the laser light to the eye of a subject.

The optical system of the laser apparatus may comprise a number of mirrors and lenses configured to guide the treatment and aiming light beams to an optical delivery fiber.

In some embodiments, the optical system comprises one or more mirrors, such as turning mirrors, for directing the treatment laser beam to a desired target site on the retina. At least one of the one or more of the mirrors are coated with a coating such that light having the first target wavelength is predominantly reflected by the at least one mirror, and light having wavelengths within the first and second suppressing ranges is predominantly transmitted by the at least one mirror.

By coating one or more mirrors in the optical system with a suitable coating that is reflective for the target wavelength, wavelength components of the treatment laser beam at the target wavelength will be passed through the laser apparatus and arrive at the desired position in the subject's eye. Conversely, since the one or more mirrors transmit light with wavelengths within the suppressing ranges, most of the light within those wavelength ranges will be dispersed, and will not be directed towards the patient, and thus not be seen by the treatment professional.

In some embodiments, the reflectivity coefficient of the at least one coated mirror at the first target wavelength is more than 97%, and the reflectivity coefficient of the at least one coated mirror within the first and second suppressing ranges is 20% or less.

It is important to deliver as much power as required from the treatment light source to the subject's eye, and consequently the more reflective the one or more mirrors are for the target wavelength, the less of the output power of the treatment laser is lost. On the other hand, the less reflective the one or more mirrors are for light with wavelengths within the suppressing ranges, the more unwanted light will be scattered before reaching the treatment professional.

In some embodiments, the laser apparatus, alternatively or additionally to the one or more coated mirrors, comprises one or more filters having high transmittance at the first target wavelength, and low transmittance within the first and second suppressing ranges.

Adding an extra optical element in the form of a blocking filter adds to the complexity of the laser apparatus; however, it can block more of the unwanted spontaneously emitted light.

In some embodiments, the first target wavelength is between 512-580nm, preferably between 512-535nm. The first target wavelength is defined as the wavelength at which most optical power is emitted. The Full Width Half Maximum (FWHM) spectral width of the diode laser output is typically 2-4 nm.

In order to effectively suppress the unwanted flashback light, the suppressing wavelength ranges should be wide enough to cover the spontaneous emission profiles of direct diode lasers. The spectral width of the spontaneous emission depends on many factors, including substrate material and quantum well design. For most direct diode lasers, the full spectral width is typically around 50 nm.

In some embodiments, the first suppressing wavelength range is at least 10 nm wide and has an upper bound within a wavelength interval between 10-20nm below the first target wavelength.

In some embodiments, the second suppressing wavelength range is at least 10 nm wide and has a lower bound within a wavelength interval between 10-20nm above the first target wavelength.

In some embodiments, when an additional external safety filter is used in the viewing path between the eye to be treated and the physician, the safety filter blocking range of said safety filter is preferably between the first target wavelength minus 15 nm and first target wavelength plus 15 nm.

In general, the first and/or second suppressing ranges should preferably overlap, or at least border, the lower and upper bounds of the safety filter blocking range, respectively. Suppressing light within the above first and second suppressing ranges from leaving the laser apparatus, means that green light flash-back to the doctor is minimized.

In some embodiments, the laser apparatus is configured to be attachable to a slit lamp and/or a slit-lamp adapter.

Some slit lamps are built to accommodate stand-alone ophthalmic laser apparatus, such as those described with respect to this invention, in which a slit-lamp adapter is used. The ophthalmic apparatus may make use of the laser apparatus of this disclosure.

Some slit lamps can be built to accommodate an integrated laser, in which the ophthalmic apparatus in embedded into the slip lamp. The ophthalmic apparatus may make use of the laser apparatus of this disclosure.

In some embodiments, the laser apparatus is configured to be attachable or integrated in a head-mounted ophthalmic treatment device.

The laser apparatus of the present disclosure may be attached to, or integrated in, a head-mounted device such as a Laser Indirect Ophthalmoscope (LIO), so that the doctor can walk around the patient during the treatment.

In another aspect, disclosed herein is a photothermal ophthalmic treatment system comprising:
- a laser apparatus according to embodiments of this disclosure;
- a microscope; and
- a safety filter configured for placement in a viewing optical path between an eye of the subject to be treated by the treatment laser beam and an eye of a physician operating the photothermal ophthalmic treatment system.

In some embodiments, the safety filter is designed to have a narrow blocking range around the treatment laser wavelength, to maximise the color clarity.

Safety filters used in slit lamps or head-mounted ophthalmic treatment devices, typically block light within a wavelengths range of between 25-35 nm, centered around the first target wavelength of the treatment laser. For a 520 nm treatment laser wavelength, this is between ~505-535nm. For other treatment laser wavelengths, for example 532nm or 577nm, a similar safety filter blocking range between 25-35nm centered around the first target wavelength would be used.

By providing a safety filter blocking only a narrow wavelength range around the treatment beam wavelength, the color clarity perceived by the doctor will be high, while the coated mirror(s) of the laser apparatus prevents unwanted flash-back light from reaching the doctor.

The safety filter may be a part of the microscope or it may be placed in the viewing optical path between a target area and a viewing input of the microscope or between a viewing output of the microscope and an eye of the physician.

In some embodiments, the microscope is part of a slit lamp.

In some embodiments, the microscope is part of a head-mounted device.

Adding the laser apparatus to a head-mounted device, allows the doctor to move around the patient during the treatment.

Various aspects disclosed herein seek to address the above and/or other matters or at least seek to provide an approach that may serve as an alternative to existing approaches.

In some embodiments, the coated mirror(s) or filter is part of the optical system that provides the treatment laser beam for delivery towards the eye of the patient, i.e. the coated mirror(s) or filter is placed as part of the delivery optics that directs the treatment laser light towards the eye of the patient.

In this way, the inventive aspects of this disclosure can be retrofitted to existing ophthalmic treatment systems, such as slit-lamps. For example, in some cases, the treatment laser beam is generated by a laser console placed on the table next to the slit lamp. In this case, the filter or coated mirror(s) as described above, could be placed in the slit-lamp adaptor.

According to a second aspect, disclosed herein is a laser module for a photothermal ophthalmic treatment apparatus, the laser module comprising:
- a treatment light source configured to generate a treatment laser beam having a first target wavelength and having a first output power;
- an aiming light source configured to generate an aiming light beam having a second target wavelength and having a second output power lower than the first output power;
wherein the laser module is further configured to block light having a wavelength within a first and/or second blocking range on either side of the first target wavelength from exiting the laser module.

By blocking light within a wavelength range on either side of the target wavelength, unwanted light emanating from the treatment light source, will be prevented from reaching the doctor during treatment. Blocking light in the context of this disclosure, should be understood to mean reducing the light by at least a factor of 5. Such reduction will reduce the power to a level comparable with the white light used for illuminating the eye of the patient. At this low power level, the flash-back is no longer disturbing to the doctor.

By blocking light within a wavelength range on either side of the target wavelength, unwanted light emanating from the treatment light source, will be prevented from reaching the doctor during treatment. Blocking light in the context of this disclosure, should be understood to mean reducing the light by at least a factor of 2, but the light should preferably be reduced by a factor of 5 or more. Such reduction will reduce the power to a level comparable with the white light used for illuminating the eye of the patient. At this low power level, the flash-back is no longer disturbing to the doctor.

Details of embodiments described above with respect to the first aspect, are also relevant and can be combined with, this second aspect.

### Brief description of the drawings

The above and other aspects will be apparent and elucidated from the embodiments described in the following with reference to the drawing in which:
FIG. 1 schematically illustrates a laser module according to embodiments of this disclosure, attached to a slit lamp device.
FIG. 2 schematically illustrates a laser module according to embodiments of the present disclosure, attached to, or integrated in a head-mounted ophthalmic treatment device.
Fig. 3 is a schematic view of the components that make up a laser module according to embodiments of this disclosure.
Fig. 4a) shows a stylized emission spectrum from a direct diode laser according to embodiments of this disclosure.
Fig. 4b) shows shows first and second suppressing ranges overlayed on the emission spectrum of the direct diode laser.
Fig. 4c) shows shows a schematic view of the reflectivity levels for the 3 ranges: first and second suppressing ranges and high reflectivity range. Overlayed with this is the emission spectrum of the direct diode laser.
Fig. 5 is a schematic view of the components that make up a laser module according to embodiments of this disclosure.
Fig. 6 shows transmission data for a spectral filter according to embodiments of this disclosure.

### DETAILED DESCRIPTION OF THE PREFERRED EMBODIMENTS

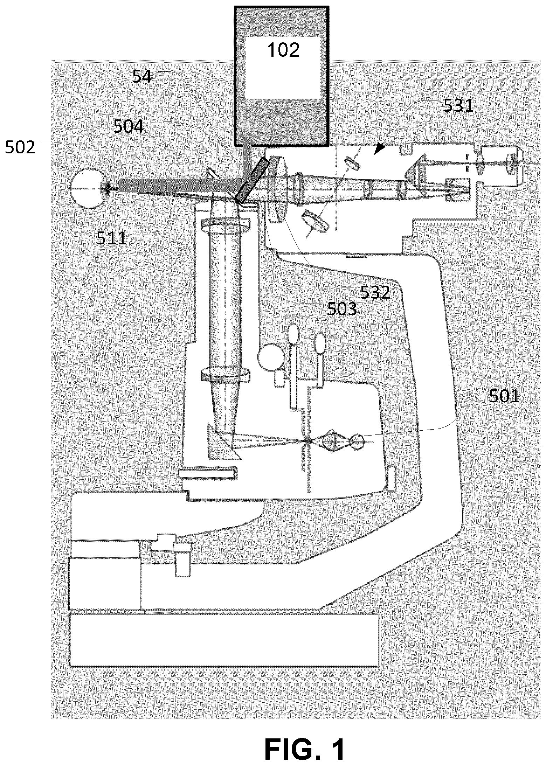

Fig. 1 schematically shows a slit lamp device 101 to which the present invention is applicable. The figure schematically shows the laser apparatus 102 attached to or incorporated into the slit lamp 101. The slit lamp 101 includes a magnifying optical device 531, such as a microscope or zoom telescope, configured to receive light at a viewing input 532 along a viewing path 503 from a target area. The central part of the slit lamp 101 includes a white light source 501 that is used to illuminate a target area in the eye 502 of the patient. This white light is directed, by means of a mirror 504, onto an illumination output path 511 that coincides with the optical viewing path 503 of the operator at the designed focal point of the diagnostic instrument at the target area. In the same fashion, the light 54 from the laser apparatus is directed towards the target area along a treatment/aiming beam path such that it coincides with the viewing path, at least at the target area.

In one embodiment, the laser apparatus 102 is attached to an existing (e.g. legacy) slit lamp device 101 via a slit-lamp adapter. The laser apparatus 102 may include a laser module, where the laser module comprises the components such as coated mirror(s), spectral filter etc. described above.

In another embodiment, the laser apparatus 102 is fully integrated into the slit lamp device 101.

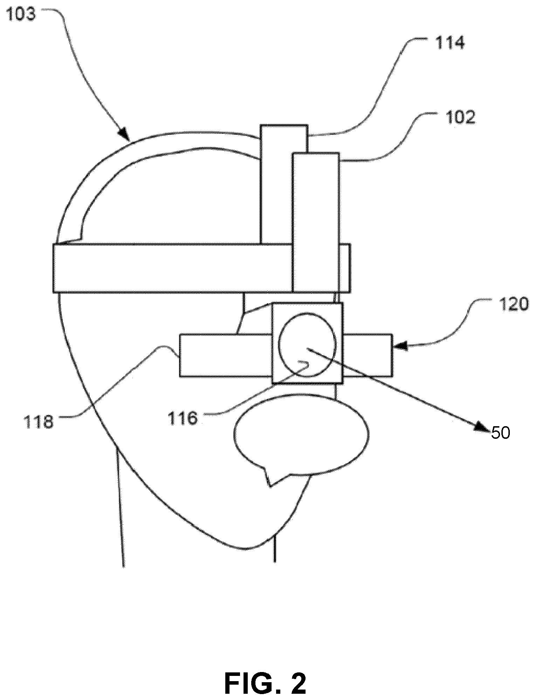

Fig. 2 is an illustration of laser treatment system according to an embodiment in which the laser apparatus 102 is attached to, or integrated in, a body-mounted LIO system. The laser apparatus 102 may be in the form of a laser module, comprising the components such as coated mirror(s), spectral filter etc. described above. In general, the body-mounted LIO system delivers the patterned laser energy to an eye of a patient, in the direction 50. A user of the LIO system is typically a physician such as an ophthalmologist. The body-mounted LIO system includes a binocular indirect ophthalmoscope 120, which is an optical device for examining the inside of the eye of the patient. The binocular indirect ophthalmoscope 120 includes an illumination unit 114 for providing white light and an optical system including a viewing aperture 118 and an exit aperture 116 from which the laser energy is emitted (which is also an entrance aperture for image information e.g. for viewing the patient's eye).

The body-mounted LIO system includes a wearable assembly 103, which secures the body-mounted LIO system, including the laser apparatus 102, to the user's body via one or more wearable objects such as a headset, a utility belt, or a backpack, among other examples. In the illustrated example, the wearable assembly 103 comprises only a headset, which is worn on the user's head.

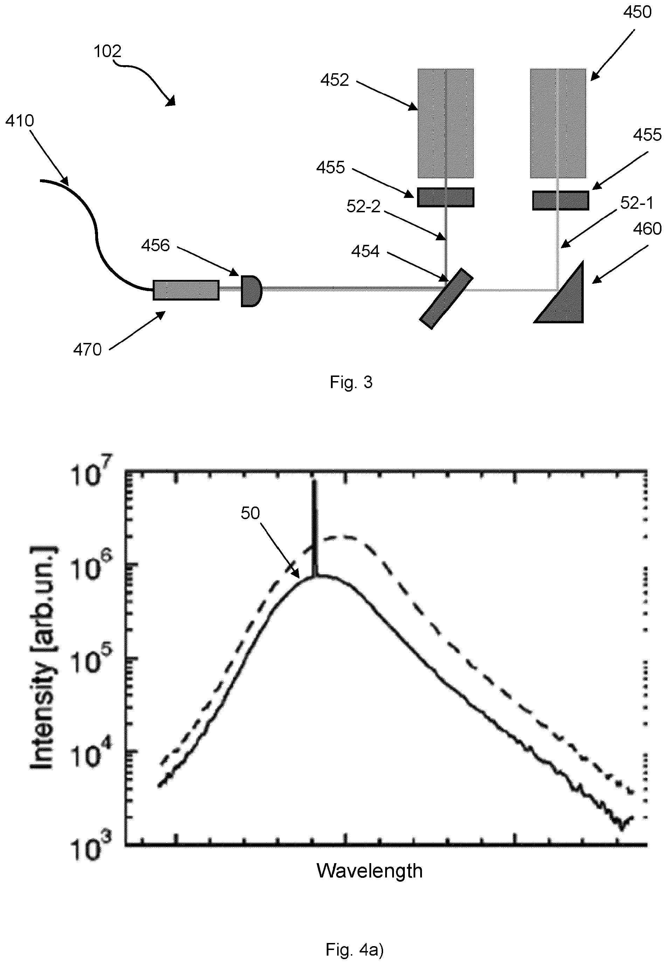

Fig. 3 is a schematic diagram of the optical layout of the laser apparatus 102 showing the main optical components that make up the laser apparatus.

The laser apparatus 102 includes a treatment laser diode 450, an aiming laser diode 452, combiner 454, and lenses 455. The laser apparatus further includes a mirror 460, which may be in the form of a turning mirror.

The treatment laser beam 52-1 produced by the treatment laser diode 450 and the aiming laser beam 52-2 produced by the aiming laser diode 452 are combined by the combiner 454, which reflects one of the beams while transmitting the other, resulting in both propagating in the same direction. The combiner may be in the form of a spectral combiner or a polarizing beam splitter. The combiner 454 directs the treatment laser beam and/or the aiming laser beam through the focusing lens 456 to the fiber optic cable 110 via the fiber optic connector 470.

This arrangement of the components of the laser apparatus 102, including the mirrors 460 for redirecting the beam allows for a compact design of the laser apparatus 102.

The mirror 460 may be coated using a suitable coating such that the mirror has a high reflectivity coefficient in a narrow wavelength band around the treatment laser wavelength such that substantially all of the treatment laser light is reflected, while having high transmission of light in the spectral regions on either side of the treatment laser wavelength. By transmitting rather than reflecting light, such light will be scattered and lost inside the laser apparatus, and will not be emitted by the laser system.

These suitable coatings can be realized such that the reflectivity coefficient of the treatment laser light is near 100%, whereas spectral regions on either side will have reflectivity of 20% or less, preferably lower than 10%, preferably lower than 5%. This will reduce the flash-back by a factor of 5, or 10, or 20.

Coating one or more mirrors in the laser apparatus as described above, has the advantage that the implementation does not significantly increase the complexity or time needed in manufacturing the laser apparatus, since the number of optical elements is preserved. Furthermore, coating one or more mirrors does not add any components such as blocking filters to the laser apparatus. Adding a blocking filter could have the unwanted effect of reducing the output intensity of the treatment laser beam.

Another advantage of coating one or more mirrors according to this disclosure, is that the mirrors can be retrofitted to existing laser apparatuses. By replacing one or more mirrors in an existing laser apparatus with one or more coated mirrors according to this disclosure, the cost will be much lower than manufacturing a new laser apparatus.

It will be appreciated that the laser apparatus 102 may include more components than shown in this illustrative example. For example, the optical system of laser apparatus 102 may comprise a number of further components, such as further mirrors, lenses, beam splitters etc.

Figure 4a) shows a stylized emission spectrum 50 from a green direct diode laser as described above with respect to laser apparatus 102. When using direct diode lasers to generate the treatment beam, the light emitted consists of two distinct portions, the laser light used for the treatment beam, and spontaneous emitted light. As can be seen from the figure, the intensity of the laser light is typically several orders of magnitude higher than the intensity of the spontaneously emitted light. The diode lasers are manufactured and selected such that the laser light falls within the reflected spectral region of the safety filter, which is external to the laser apparatus. However, the spontaneous emitted light has wavelength components that are typically outside this spectral region. Although this spontaneous emitted light is not coherent laser light, and the optical power is orders of magnitude lower than the laser light, some treatment professionals may find this light distracting or disturbing. In the current example of a target treatment beam wavelength in the green part of the spectrum, any flashes of light appearing green to the doctor should therefore be avoided.

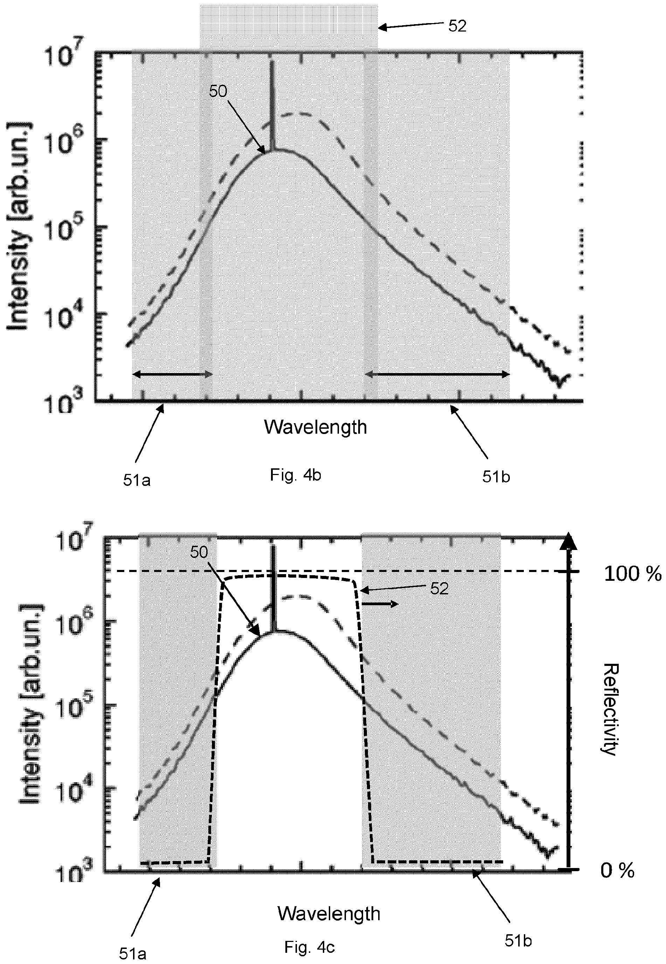

Fig. 4b) shows the first and second suppressing ranges 51a and 51b overlayed on the emission spectrum 50 of the direct diode laser. The figure further illustrates the blocking range 52 of the external safety filter. The first suppressing range 51 a should be chosen such that the upper bound of the range overlaps, or at least borders on, the lower bound of the safety filter blocking range 52. Likewise, the second suppressing range 51b should be chosen such that the lower bound of the range overlaps, or at least borders on, the upper bound of the safety filter blocking range 52. This ensures that as much as possible of the spontaneously emitted light from the treatment laser beam is prevented from reaching the doctor. Safety filters used in slit lamps or head-mounted ophthalmic treatment devices, typically block light within a wavelengths range of 30 nm, centered around the first target wavelength of the treatment laser. For a 520 nm treatment laser wavelength, this corresponds to 505-535nm. In this example, for a 520nm treatment laser wavelength, the first suppressing range is 490-508nm, and the second suppressing range is 532-550nm.

As can be seen from the figure 4c, first and second suppressing ranges having widths of ~20nm, means that the coated mirror reflects less than 20% and thereby transmits more than 80% of the spontaneously emitted light. As a result, a majority of the spontaneously emitted light will be dispersed within the laser apparatus, and never reach the doctor. Depending on the desired effectiveness of preventing the unwanted spontaneously emitted light from reaching the doctor, these suppressing ranges could be made wider or narrower as appropriate. As an example, in prior art systems, in order to avoid the unwanted flash-back light in the 490-508 and 532-550nm intervals, the external safety filter would also have to block these wavelengths. This would have the disadvantage that the color perceived by the doctor when looking at the patient's eye using the white light source of the slit lamp or LIO, would be distorted. By applying coated mirrors according to this disclosure, narrower safety filters can be used, maintaining high color clarity.

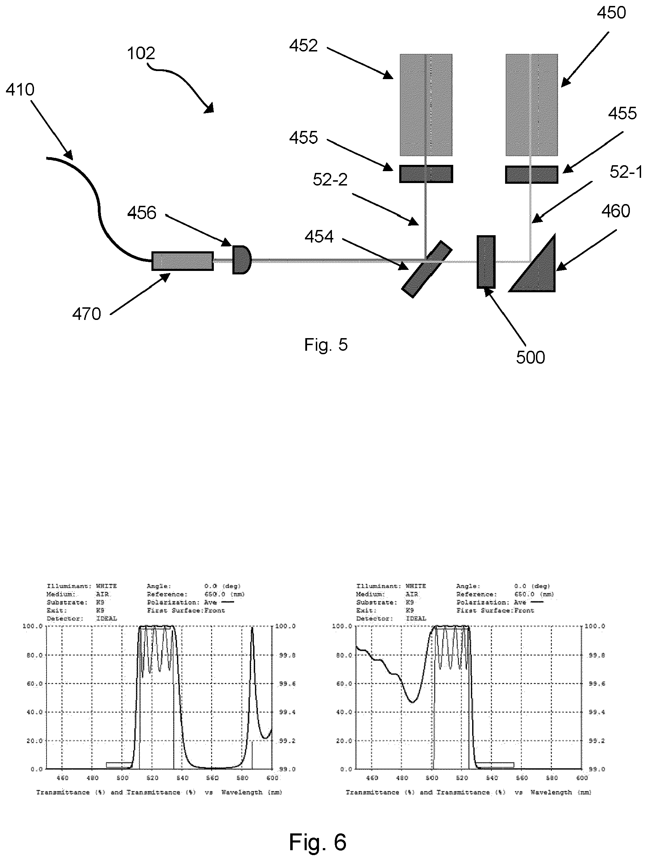

Turning now to figure 5, another embodiment of the laser apparatus 102 is shown. In this embodiment, as an alternative, or in addition to, coating mirror 460, the optical system of laser apparatus 102 may include an optical element 500, such as a spectral filter, in the optical path configured to block light in the desired spectral regions on either side of the treatment wavelength. This will reduce or eliminate the unwanted flash-back light from reaching the doctor, but may also reduce the intensity of the treatment laser beam. This reduction in intensity of the treatment beam may up to 3% or more, which may be an unacceptably high level.

Fig. 6 shows the transmittance of the filter 500 described with respect to fig. 5 above, as a function of the wavelength of the incident light. Transmittance is on average above 97% in the target treatment beam wavelength interval between 512-525nm, and reflectivity is above 95% for the suppressing ranges 490-507nm and 530-555nm respectively. Adding this filter to the laser apparatus, will disperse the unwanted spontaneously emitted light, while allowing the treatment beam to pass. As explained above with respect to figure 5, this spectral filter can be added to the laser apparatus either as a replacement for coating one or more mirrors, or in addition to coating one or more mirrors with a coating as previously described.

Embodiments of the method described herein can be implemented by means of hardware comprising several distinct elements. In the apparatus claims enumerating several means, several of these means can be embodied by one and the same element, component or item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, elements, steps or components but does not preclude the presence or addition of one or more other features, elements, steps, components or groups thereof.

Various aspects disclosed herein may be summarized by the following items:
1. A laser apparatus for providing a treatment laser beam for photothermal ophthalmic treatment, the laser apparatus comprising:
   - a treatment light source configured to generate a laser beam having a first target wavelength and having a first output power;
   - an aiming light source configured to generate an aiming light beam having a second target wavelength and having a second output power lower than the first output power;
   wherein the laser apparatus is configured to selectively suppress a set of wavelength components of the laser beam generated by the treatment light source to create a treatment laser beam, the set of wavelength components having wavelengths within a first and/or second suppressing wavelength range, the first and/or second suppressing wavelength ranges being located on respective sides of the first target wavelength, and wherein the laser apparatus is further configured to output the treatment laser beam.
2. The laser apparatus according to item 1, wherein the first output power is between 100 mW - 3 W.
3. The laser apparatus according to any of the preceding items, wherein the second output power of the aiming light source is between 1 µW and 1 mW.
4. The laser apparatus according to any of the preceding items, wherein the treatment light source comprises one or more direct diode lasers for generating the treatment laser beam.
5. The laser apparatus according to any of the preceding items, wherein the laser apparatus comprises an optical system for delivering the treatment laser beam and the aiming light beam to an optical fiber.
6. The laser apparatus according to the preceding item, wherein the optical system comprises one or more mirrors, and at least one of the one or more mirrors are coated with a coating such that light having the first target wavelength is reflected by the at least one mirror, and light having wavelengths within the first and second suppressing wavelength ranges is transmitted by the at least one mirror.
7. The laser apparatus according to the preceding item, wherein the reflectivity coefficient of the at least one coated mirror at the first target wavelength is more than 90%, preferably more than 95%, most preferably more than 97%, and the reflectivity coefficient of the at least one coated mirror within the first and second suppressing wavelength ranges is 20% or less.
8. The laser apparatus according to any of the preceding items, wherein the one or more coated mirrors is a turning mirror.
9. The laser apparatus according to any of the preceding items, wherein the laser apparatus comprises one or more filters to suppress light within the first and/or second wavelength ranges.
10. The laser apparatus according to any of the preceding items, wherein the first target wavelength is between 512-580nm.
11. The laser apparatus according to any of the preceding items, wherein the first target wavelength is between 512-535 nm.
12. The laser apparatus according to any of the preceding items, wherein the first suppressing wavelength range is at least 10 nm wide and has an upper bound within a wavelength interval between 10-20nm lower than the first target wavelength.
13. The laser apparatus according to any of the preceding items, wherein the second suppressing wavelength range is at least 10 nm wide and has a lower bound within a wavelength interval between 10-20nm higher than the first target wavelength.
14. The laser apparatus according to any of the preceding items, wherein the laser apparatus is an ophthalmic apparatus that is configured to be attachable to a slit lamp or slit-lamp adapter.
15. The laser apparatus according to any of the preceding items, wherein the laser apparatus is configured to be attachable to, or integrated in, a head-mounted ophthalmic treatment device.
16. The laser apparatus according to any of the preceding items, wherein the coated mirror(s) or filter is placed as part of the delivery optics that directs the treatment laser light towards the eye of the patient.
17. A photothermal ophthalmic treatment system comprising:
   - a laser apparatus according to any of items 1-16;
   - a microscope; and
   - a safety filter placed in the optical path of the treatment beam.
18. The system according to the preceding item, wherein the safety filter blocks light within a wavelength range of between 25-35nm and a center wavelength between 515-535nm.
19. The system according to item 17 or 18, wherein the microscope is part of a slit lamp.
20. The system according to item 17 or 18, wherein the microscope is part of a head-mounted device.

## Claims

1. A laser apparatus for providing a treatment laser beam for photothermal ophthalmic treatment, the laser apparatus comprising:
- a treatment light source configured to generate a laser beam having a first target wavelength and having a first output power;
- an aiming light source configured to generate an aiming light beam having a second target wavelength and having a second output power lower than the first output power;
wherein the laser apparatus is configured to selectively suppress a set of wavelength components of the laser beam generated by the treatment light source to create a treatment laser beam, the set of wavelength components having wavelengths within a first and/or second suppressing wavelength range, the first and/or second suppressing wavelength ranges being located on respective sides of the first target wavelength, and wherein the laser apparatus is further configured to output the treatment laser beam.

2. The laser apparatus according to claim 1, wherein the first output power is between 100 mW - 3 W.

3. The laser apparatus according to claim 1 or 2, wherein the second output power of the aiming light source is between 1 µW and 1 mW.

4. The laser apparatus according to any one of the preceding claims, wherein the treatment light source comprises one or more direct diode lasers for generating the laser beam output by the treatment light source.

5. The laser apparatus according to any one of the preceding claims, wherein the laser apparatus comprises an optical system for providing the treatment laser beam and, optionally, the aiming light beam for delivery towards an eye of a subject to be treated.

6. The laser apparatus according to claim 5, wherein the optical system comprises one or more mirrors, and at least one of the one or more mirrors is coated with a coating configured such that light having the first target wavelength is reflected by the at least one mirror, and light having wavelengths within the first and second suppressing wavelength ranges is transmitted by the at least one mirror.

7. The laser apparatus according to the claim 6, wherein the reflectivity coefficient of the at least one coated mirror at the first target wavelength is more than 90%, preferably more than 95%, most preferably more than 97%, and the reflectivity coefficient of the at least one coated mirror within the first and second suppressing wavelength ranges is 20% or less.

8. The laser apparatus according to claim 6 or 7, wherein the one or more coated mirrors is a turning mirror.

9. The laser apparatus according to any one of the preceding claims, wherein the optical system comprises one or more filters to suppress light within the first and/or second wavelength ranges.

10. The laser apparatus according to any one of the preceding claims, wherein the first target wavelength is between 512-580nm, in particular between 512-535 nm.

11. The laser apparatus according to any one of the preceding claims, wherein the first suppressing wavelength range is at least 10 nm wide and has an upper bound within a wavelength interval between 10-20nm lower than the first target wavelength.

12. The laser apparatus according to any one of the preceding claims, wherein the second suppressing wavelength range is at least 10 nm wide and has a lower bound within a wavelength interval between 10-20nm higher than the first target wavelength.

13. A photothermal ophthalmic treatment system comprising:
- a laser apparatus comprising a treatment light source configured to generate a laser beam having a first target wavelength and having a first output power, an aiming light source configured to generate an aiming light beam having a second target wavelength and having a second output power lower than the first output power;
- a microscope; and
- a safety filter configured for placement in a viewing optical path between an eye of the subject to be treated by the treatment beam and an eye of a physician operating the photothermal ophthalmic treatment system;
wherein the laser apparatus is configured to selectively suppress a set of wavelength components of the laser beam generated by the treatment light source to create a treatment laser beam, the set of wavelength components having wavelengths within a first and/or second suppressing wavelength range, the first and/or second suppressing wavelength ranges being located on respective sides of the first target wavelength, and wherein the laser apparatus is further configured to output the treatment laser beam.

14. The system according to claim 13, wherein the safety filter blocks light within a wavelength range of between 25-35nm and a center wavelength between 515-535nm.

15. The system according to claim 13 or 14, wherein the microscope is part of a slit lamp or of a head-mounted device.
